# EUROPÄISCHE PATENTSCHRIFT

(11) **EP 2 309 925 B1**
(45) Veröffentlichungstag und Bekanntmachung des Hinweises auf die Patenterteilung: **21.12.2016**
(21) Anmeldenummer: 09777470.7
(22) Anmeldetag: 27.07.2009
(51) Int. Cl.: A61B 6/00

(54) **RÖNTGENBILD-AUFNAHMESYSTEM UND RÖNTGENBILD-AUFNAHMEVERFAHREN ZUR AUFNAHME VON BILDDATEN MIT RÖNTGENGERÄTEN FÜR EINE VOLUMENREKONSTRUKTION**
X-RAY IMAGE RECORDING SYSTEM AND X-RAY IMAGE RECORDING METHOD FOR RECORDING IMAGE DATA USING X-RAY DEVICES FOR VOLUME RECONSTRUCTION
SYSTÈME DE PRISE D'UNE IMAGE RADIOGRAPHIQUE ET PROCÉDÉ DE PRISE D'UNE IMAGE RADIOGRAPHIQUE POUR ENREGISTRER DES DONNÉES D'IMAGES AVEC DES APPAREILS RADIOGRAPHIQUES POUR UNE RECONSTRUCTION EN VOLUME

(30) Priorität: 31.07.2008 DE 102008035736
(43) Veröffentlichungstag der Anmeldung: 20.04.2011
(73) Patentinhaber: Fraunhofer-Gesellschaft zur Förderung der angewandten Forschung e.V., 80686 München (DE)
(72) Erfinder: JANK, Emanuel, 12589 Berlin (DE); UHLMANN, Eckart, 25368 Kiebitzreihe (DE)
(74) Vertreter: Pfenning, Meinig & Partner mbB
(86) Internationale Anmeldenummer: PCT/EP2009/005437
(87) Internationale Veröffentlichungsnummer: WO 2010/012441

(56) Entgegenhaltungen:
- EP-A- 0 972 490
- EP-A- 1 852 822
- WO-A-00/66971
- US-A1- 2004 015 077

## Beschreibung

Die vorliegende Erfindung bezieht sich auf ein Röntgenbild-Aufnahmesystem (sowie auf ein entsprechendes Röntgenbild-Aufnahmeverfahren) zur Aufnahme von Röntgenprojektionsbildern und zur Aufnahme von Ausrichtungsinformationen für die aufgenommenen Röntgenprojektionsbilder. Das Aufnahmesystem bzw. das Aufnahmeverfahren können dabei insbesondere im Rahmen eines handelsüblichen C-Bogensystems realisiert werden, das dann mit geeigneten Hardware- und/oder Software-Maßnahmen für einen Betrieb als erfindungsgemäßes Röntgenbild-Aufnahmesystem ausgebildet wird.

Aus der US 2004/015077 A1 ist die Rekonstruktion von dreidimensionalen Koordinaten von Markern bekannt. Ebenso ist die Verwendung von in einer Look-Up-Tabelle abgespeicherten Information zur späteren Verwendung in der Computer-assistierten Chirurgie bekannt. Die Computer-assistierte Chirurgie der D1 wird dabei auf Basis von Röntgenprojektionsbildern durchgeführt, die mit einem C-Arm-System aufgenommen wurden.

In der Medizin dient die Bildgebung zur Darstellung von inneren Bereichen eines Patienten und wird zur Diagnose und zur Behandlungskontrolle benutzt. Während eines chirurgischen Eingriffs ist die Bildgebung mittels Röntgensystemen (zumeist C-Bögen) weit verbreitet. Bei dieser Form der Bildgebung durchdringen Röntgenstrahlen das abzubildende Gewebe und werden dabei abgeschwächt. Am Bilddetektor des Röntgensystems entsteht ein Projektionsbild des durchleuchteten Objekts, bei dem räumliche Informationen überlagert dargestellt werden. Der Informationsgehalt eines Projektionsbildes ist im Gegensatz zu dreidimensionalen Volumenbilddaten (nachfolgend auch als Schichtbilddaten einer Schichtbildrekonstruktion bezeichnet) eingeschränkt. Die exakte Kontrolle von Implantatpositionen oder die Beurteilung repositionierter Gelenkflächen nach Frakturen ist mit Projektionsbildern kaum möglich.

Die technische Herausforderung, um aus Projektionsbildern Volumenbilddaten zur rekonstruieren, liegt in der Bestimmung der dafür notwendigen Positionsinformationen und der Projektionsgeometrie. Weiterhin müssen für eine Rekonstruktion eines Schichtbildes mehrere Projektionsbilder aus unterschiedlichen Raumrichtungen aufgenommen werden. Dabei muss berücksichtigt werden, dass das zu rekonstruierende Objekt stets in den Röntgenbildern abgebildet wird (dass also der Abbildungsbereich, in dem das abzubildende Objekt positioniert ist, stets auf diese sensitive Fläche des Röntgendetektors abgebildet wird).

Zur Aufnahme von Röntgenbilddaten im Operationssaal werden 2D-Röntgengeräte (insbesondere C-Bögen) verwendet. C-Bögen bestehen aus einer C-förmigen Aufnahmeeinheit auf einer verstellbaren und fahrbaren Halterung. An den Enden des C bzw. des C-Bogens sind die Röntgenquelle (Röntgenröhre) und der Detektor befestigt. Das C kann derart an einem OP-Tisch positioniert werden, dass sich der Tisch mit dem Patienten innerhalb des C, zwischen Röntgenquelle und Detektor, und damit im Strahlengang des Geräts befindet und ein Röntgenbild des Patienten aufgenommen werden kann. C-Bögen bieten die Möglichkeit, die Aufnahmeeinheit in mehreren Rotationsrichtungen um den Patienten zu rotieren und Projektionsbilder aus verschiedenen Richtungen aufzunehmen. Eine Eigenschaft der C-Bögen ist dabei, dass der Zentralstrahl des Röntgensystems in der Regel nicht durch die Rotationsachse verläuft. Diese Bauform lässt erheblich kleinere und leichtere mechanische Bauformen zu, verursacht aber, dass bei einer Drehung des C das Objekt aus dem Bildzentrum gelangt.

Zur Aufnahme von 3D-Bilddaten wurden im Stand der Technik solche C-Bögen modifiziert, indem die einzelnen beweglichen Achsen des C-Bogens mit Messmitteln und Motoren ausgestattet wurden, um die Aufnahmeeinheit (Röhre und Detektor) auf einer Bahn um das Aufnahmeobjekt zu bewegen, dabei Röntgenbilder aufzunehmen und die Position der Bilder zu bestimmten um daraus 3D-Bilddaten rekonstruieren zu können. Beispielsweise ist das System Ziehm Vario 3D aus dem Stand der Technik bekannt. Dieser 3D-C-Bogen basiert auf einer Standard-C-Bogen-Mechanik, die mit zusätzlichen Encodern und Motoren ausgestattet ist. Das System bietet eine automatische Bewegung des C um den Patienten mit automatischer Bildaufnahme. Um das Objekt im Bildzentrum zu halten, werden die horizontalen und vertikalen Achsen des Systems parallel zur C-Bewegung nachgeregelt. Die Rotation erfolgt um 135 Grad und bietet im Anschluss eine Volumenrekonstruktion.

Die aus dem Stand der Technik bekannten Systeme weisen insbesondere den Nachteil auf, dass mehrere Sensoren und Motoren und gegebenenfalls eine Vorrichtung zur automatischen Ausrichtung des Röntgensystems fest in den C-Bogen integriert werden müssen. Der mechanische Aufwand zu einer solchen Ausrüstung eines Röntgensystems ist damit teuer.

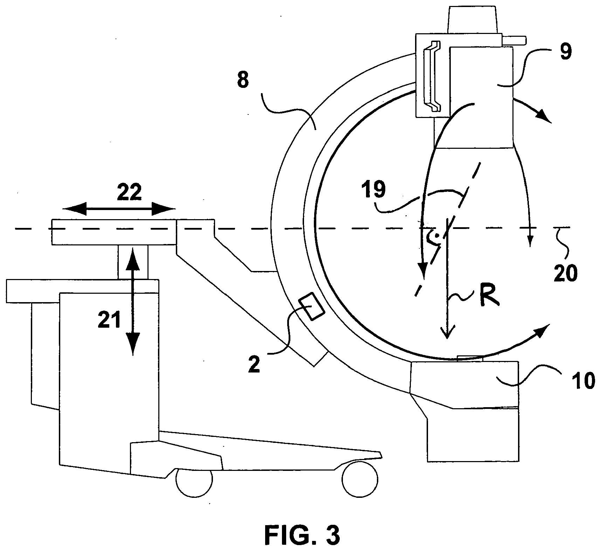

Darüber hinaus ist bei den bekannten Geräten die Bewegungsfreiheit im 3D-Aufnahmemodus auf eine Rotationsrichtung (C-Achse 19 oder Propellerachse bzw. P-Achse 20, siehe z.B. Figur 3) eingeschränkt. Dadurch geht die Flexibilität des Röntgensystems teilweise verloren. Die Bildaufnahme kann der klinischen Fragestellung und der gewünschten Rekonstruktionsqualität so nicht flexibel angepasst werden.

Schließlich bieten die bekannten Systeme keine Möglichkeit, das Rekonstruktionsergebnis durch eine gezielte Aufnahme weiterer Bilder zu verbessern. Nach Abschluss der Bildaufnahme und Betrachtung des Rekonstruktionsergebnisses ist nicht erkennbar, aus welchen Richtungen weitere Bilder aufgenommen werden müssen, um die Qualität der 3D-Rekonstruktion zu verbessern.

Aufgabe der vorliegenden Erfindung ist es somit, ein Röntgenbild-Aufnahmesystem und ein Röntgenbild-Aufnahmeverfahren zur Verfügung zu stellen, mit dem auf einfache, wirtschaftliche Art und Weise mit einem einfachen mechanischen Aufbau (insbesondere dem mechanischen Aufbau eines Standard-C-Bogensystems) aus unterschiedlichen Richtungen Röntgen-Projektionsbilder von einem Objekt aufgenommen werden können und mit dem, durch Bestimmung der Lage der einzelnen Projektionsbilder im Raum (im Moment ihrer Aufnahme) alle notwendigen Daten (Positionsdaten) zur Rekonstruktion von Schichtbildern aus den aufgenommenen Röntgen-Projektionsbildern mit hoher Genauigkeit bestimmt werden können.

Diese Aufgabe wird durch ein Röntgenbild-Aufnahmesystem gemäß Patentanspruch 1 sowie durch ein Röntgenbild-Aufnahmeverfahren gemäß Patentanspruch 11 gelöst. Vorteilhafte Ausgestaltungsformen des erfindungsgemäßen Aufnahmesystems bzw. Aufnahmeverfahrens lassen sich jeweils den abhängigen Ansprüchen entnehmen.

Nachfolgend wird ein erfindungsgemäßes Aufnahmesystem (und somit auch Aufnahmeverfahren) zunächst allgemein beschrieben. Dem schließt sich ein ausführliches konkretes Ausführungsbeispiel für die Realisierung des erfindungsgemäßen Aufnahmesystems an.

Die einzelnen Merkmale des speziellen Ausführungsbeispiels müssen dabei nicht in der gezeigten Kombination verwirklicht werden, sondern können im Rahmen der vorliegenden Erfindung auch in beliebigen anderen Kombinationen realisiert sein.

Grundlegende Idee der vorliegenden Erfindung ist es, diejenigen Positionsdaten eines jeden Röntgen-Projektionsbildes, das zur Bildrekonstruktion von Schichtbildern herangezogen wird, nicht über mehrere, fest in die Aufnahmeeinheit integrierte Sensoren/Motoren zu erfassen bzw. zu berechnen, sondern diese notwendigen Positionsdaten auf Basis der Verwendung eines einzelnen Lagesensors abzuleiten. Dieser Lagesensor (wie nachfolgend beschrieben, kann es sich dabei beispielsweise um einen Sensor handeln, der die Lage des Aufnahmesystems relativ zum Beschleunigungsvektor der Erdbeschleunigung bestimmt) bestimmt für jedes zur Rekonstruktion herangezogene Projektionsbild im Moment der Aufnahme dieses Projektionsbildes die momentane Ausrichtung des Systems aus Röntgenröhre und Röntgendetektor relativ zu einer Referenzrichtung (also beispielsweise der Erdbeschleunigungsrichtung).

Für jedes aufgenommene, zur späteren Bildrekonstruktion herangezogene Projektionsbild wird dann die zugehörige, durch den Lagesensor erfasste momentane Ausrichtung von Röntgenröhre und Röntgendetektor im Moment der Aufnahme des Projektionsbildes zusammen mit dem jeweiligen Röntgen-Projektionsbild abgespeichert, so dass hier eine eindeutige Zuordnung von Ausrichtung und Röntgen-Projektionsbild gegeben ist. Wie nachfolgend noch im Detail beschrieben, werden dann für jedes zur Rekonstruktion verwendete Projektionsbild die notwendigen Positionsdaten aus der abgespeicherten, zugehörigen Ausrichtung berechnet. Die Positionsdaten sind dabei diejenigen Daten, die die Lage des aufgenommenen Projektionsbildes und die Lage der Röntgenröhre in diesem Moment im Raum so beschreiben, dass anhand dieser Daten und des zugehörigen Röntgen-Projektionsbildes eine Schichtbildrekonstruktion in ausreichender Genauigkeit möglich ist. Die Kalibrierung und die erforderlichen Positionsdaten werden nachfolgend noch detailliert beschrieben (im Einzelnen sind dies die Position/Orientierung des Bildkoordinatensystems relativ zu einem Basiskoordinatensystem BKS (unbeweglich während der Anwendung/Kalibrierung), die Skalierung des Bildes (Größe eines Bildpunktes) und die Position der Röntgenquelle relativ zum BKS oder zum Bild.)

Die Umrechnung aus den mit Hilfe des Lagesensors bestimmten Ausrichtungen in die notwendigen Positionsdaten kann beispielsweise in einer Recheneinheit des Systems geschehen. Ebenso ist es jedoch auch denkbar, dass die abgespeicherten Röntgen-Projektionsbilder samt zugehöriger momentaner Ausrichtungen z.B. mit Hilfe einer tragbaren Festplatte an ein externes Rechensystem (PC oder Ähnliches), übergeben werden.

Besonders bevorzugt geschieht dabei, wie ebenfalls nachfolgend noch näher beschrieben wird, die Umrechnung bzw. Transformation von Ausrichtungsdaten in notwendige Positionsdaten mit Hilfe einer Vorab-Kalibrierung des Systems. Bei einer solchen Kalibrierung können für verschiedene Stellungen des Röhren-Detektorsystems einerseits mit Hilfe des Lagesensors die zugehörigen Ausrichtungen erfasst werden und andererseits kann mit Hilfe einer externen Kalibriereinheit eine Bestimmung der zugehörigen notwendigen Positionsdaten vorgenommen werden. Der bestimmte Zusammenhang zwischen notwendigen Positionsdaten und Ausrichtungsdaten bzw. Ausrichtung kann dann beispielsweise in Form einer Look-Up-Tabelle (LUT) in einem Speicher abgelegt werden, so dass dann später während des Betriebs des Aufnahmesystems (und nach Entfernung der externen Kalibriereinheit) bestimmte Ausrichtungswerte mit Hilfe der LUT eindeutig (oder nahezu eindeutig) in die zugehörigen notwendigen Positionsdaten umrechenbar sind.

Eine solche Kalibriereinheit kann beispielsweise ein Positionsmesssystem aufweisen (z.B. Positionskamera), mit der ein ortsfest am Röntgendetektor angebrachter dreidimensionaler Kalibrierkörper hinsichtlich seiner Lage und Ausrichtung im Raum auswertbar ist (beispielsweise auf optischem Wege mit nachgeschalteter Bildverarbeitung). Da der Kalibrierkörper fest am Detektor angeordnet ist, kann aus der Bestimmung der Lage/Ausrichtung des Kalibrierkörpers eineindeutig auf die Lage und Ausrichtung des Detektors (und somit auf die Lage des Röhren-Detektorsystems) geschlossen werden. Die so gewonnenen Positionsdaten bezüglich der Lage des Röhren-Detektorsystems werden dann mit den gleichzeitig erfassten Ausrichtungsdaten des Lagesensors wie vorbeschrieben in Form einer Kalibriertabelle bzw. LUT abgespeichert. Während des eigentlichen Aufnahmebetriebes (in dem dann kein Kalibriersystem mehr vorhanden ist, sondern sich nur noch die Kalibriertabelle im Speicher befindet), können dann für jedes zur Bildrekonstruktion heranzuziehende Projektionsbild im Moment dessen Aufnahme die zugehörige Ausrichtung mit dem Lagesensor bestimmt werden und, beispielsweise mit Hilfe eines Interpolationsverfahrens, aus der entsprechende Stützstellen abspeichernden LUT die zugehörigen notwendigen Positionsdaten bestimmt werden.

Ein besonderer Vorteil der erfindungsgemäßen Definition genau einer Referenzrichtung (auf die sich die Ausrichtungsdaten beziehen) ist es, dass mit Hilfe eines einfachen, gegebenenfalls auch nachträglich anbringbaren Lagesensors alle notwendigen Daten (z.B. während der vorbeschriebenen Kalibrierung) mit hoher Genauigkeit erfasst werden können.

In einer weiteren vorteilhaften Ausgestaltungsform weist das erfindungsgemäße System eine Hinweiseinheit auf, mit der auf Basis der zugehörigen Ausrichtungen bereits aufgenommener Röntgen-Projektionsbilder mit Hilfe geeigneter Algorithmen vom System weitere Ausrichtungen des Systems Röntgenröhre-Röntgendetektor berechnet werden können und ausgegeben werden können, unter denen für eine optimale Bildrekonstruktion noch weitere Röntgen-Projektionsbilder aufgenommen werden müssen. Die weiteren Ausrichtungen bzw. Richtungen, aus denen noch Röntgen-Projektionsbilder vom abzubildenden Objekt gemacht werden müssen, können auf Basis der bereits aufgenommenen Projektionsbilder berechnet werden.

Die vorliegende Erfindung schlägt somit ein System vor, bei dem basierend auf den Daten eines Sensorsystems zur Messung einer Referenzrichtung (z.B. Schwerkraftsensor) die Bestimmung der räumlichen Eigenschaften aufgenommener Röntgenbilder, die Erfassung der Abbildungseigenschaften des Röntgengeräts und die Rekonstruktion von Volumenbilddaten sowie eine Benutzerführung durchgeführt werden können. Ein besonderer Vorteil dieses Systems ist die Möglichkeit der einfachen Integration dieser 3D-Bildgebungsfunktion (in Software und/oder Hardware) in vorhandene Röntgengeräte (insbesondere C-Bogen-Röntgensysteme), ohne mechanische Veränderungen vornehmen zu müssen.

Der Lagesensor wird dabei fest mit dem Aufnahmegerät (also der Einheit aus Röntgenröhre und Detektor) verbunden. Durch das Auslesen der Sensormesswerte kann die Orientierung der Röntgenröhre und des Röntgendetektors relativ zur vordefinierten Referenzrichtung (Erdschwererichtung) bestimmt werden. Da jede Änderung der Ausrichtung des Röntgenaufnahmesystems eine messbare Änderung der Richtungsdaten verursacht, können den Richtungsdaten Positionsinformationen zugeordnet werden. Die Parameter der dazu benötigten Transformation bzw. Zuordnungsvorschrift können durch einen Kalibrierprozess bestimmt werden. Nachdem die räumliche Lage für jedes Röntgen-Projektionsbild bestimmt ist, kann dann schließlich die Volumenrekonstruktion bzw. die Schichtbildrekonstruktion durchgeführt werden. Darüber hinaus ist es möglich, Hinweise für eine optimale Benutzung des Systems zu berechnen und anzuzeigen.

Das erfindungsgemässe System weist einen Lagesensor zur Messung der Referenzrichtung relativ zur Röntgenaufnahmeeinheit, eine Recheneinheit zur Umrechnung der Richtungsdaten bzw. Ausrichtungsdaten in Positionsdaten, eine Rekonstruktionseinheit zur Berechnung von Volumenbilddaten aus den Projektionsbildern und den Projektionsdaten, sowie bevorzugt eine Benutzerschnittstelle zur Darstellung der Bilddaten und zur Interaktion mit dem Benutzer auf. Die Sensoreinheit misst dabei bevorzugt die Richtung der Erdbeschleunigung und ist fest in das Röntgengerät integriert bzw. an ihm angeordnet. Darüber hinaus generiert bevorzugt die Software Hinweise zur Bedienung und Ausrichtung des Röntgengerätes mit dem Ziel, für die Rekonstruktion optimale Bilddaten in optimaler Bildlage aufzunehmen.

Um horizontale und vertikale Bewegungen zu messen, welche keinen Einfluss auf die Ausrichtung des Systems zum Erdschwerefeld haben, kann gegebenenfalls der beschriebene Lagesensor verwendet werden oder es können zusätzliche Sensoren benutzt werden, um solche Bewegungen zu erfassen. Die Erfassung dieser zusätzlichen translatorischen Bewegungen kann direkt erfolgen (z.B. mit Abstands-, Positionssensoren) oder über die Auswertung von Beschleunigungsdaten (zweifache Integration der Beschleunigung über die Zeit ergibt den zurückgelegten Weg).

Neben den bereits vorbeschriebenen Vorteilen weist die vorliegende Erfindung gegenüber den aus dem Stand der Technik bekannten Systemen vor allen Dingen die folgenden Vorteile auf:
- Es wird ein einfaches Konzept zur Nachrüstung bestehender Röntgenaufnahmesysteme mit einer 3D-Funktion zur Verfügung gestellt.
- Dabei sind keine mechanischen Änderungen am Gerät selbst notwendig.
- Das System führt zu keinen Einschränkungen in den Bewegungsmöglichkeiten des Aufnahmegerätes durch die vorbeschriebene Erweiterung.
- Alle Bewegungsachsen sind somit für die Aufnahme von Projektionsbilddaten, die zur Rekonstruktion herangezogen werden können, nutzbar.

Nachfolgend wird die Erfindung nun anhand eines ausführlichen Ausführungsbeispiels beschrieben.

Dabei zeigen:
- Figur 1: die Grundkonfiguration des Röntgenbild-Aufnahmesystems gemäß diesem Ausführungsbeispiel;
- Figur 2: die Anbringung und das Nutzungsprinzip des verwendeten Lagesensors;
- Figur 3: die einzelnen Bewegungsachsen des beispielhaften Aufnahmesystems;
- Figur 4: das Prinzip der Kalibrierung des Aufnahmesystems gemäß Figur 1;
- Figur 5: während einer Kalibrierung ermittelte Stützstellen und daraus interpolierte Stützstellen einer beispielhaften Kalibriertabelle (LUT);
- Figur 6: die Systemkomponenten des Systems aus Figur 1 während der Kalibrierung;
- Figur 7: den Datenfluss der Softwarekomponenten des Beispielsystems aus Figur 1 während der Kalibrierung und während der Rekonstruktionsphase.

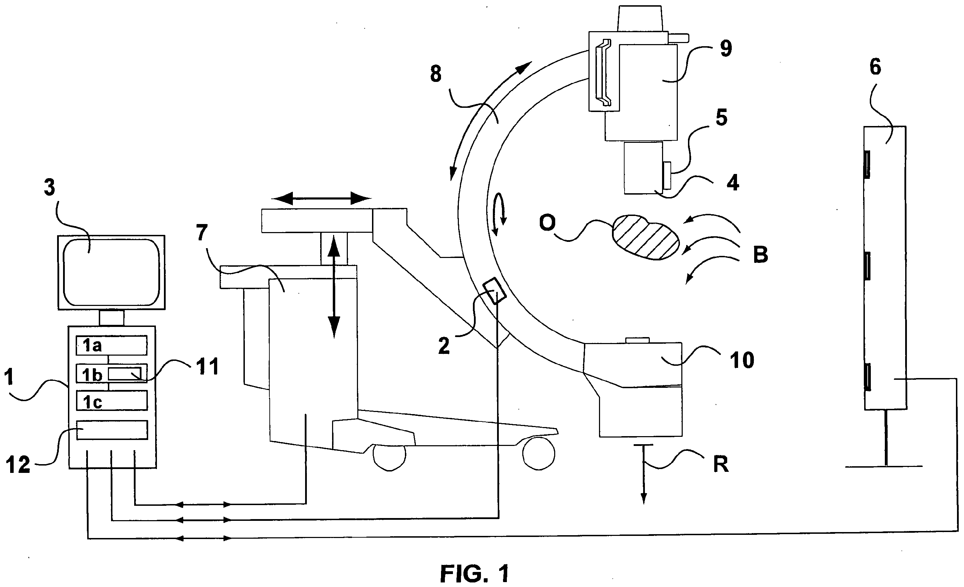

Figur 1 zeigt ein Röntgenbild-Aufnahmesystem gemäß der vorliegenden Erfindung in einem ersten Ausführungsbeispiel. Das Röntgenbild-Aufnahmesystem ist auf Basis eines Standard-C-Bogens aufgebaut. Der C-Bogen 8 trägt an seinem ersten Ende einen Röntgenbilddetektor 9 (hier ein analoger Detektor in Form eines Röntgen-Bildverstärkers BV, es kann sich jedoch auch um einen digitalen Flachbilddetektor handeln) und an seinem zweiten, gegenüber liegenden Ende die Röntgenröhre 10. Im Zentrum des C bzw. zwischen den beiden Enden des C liegt zwischen der Röntgenröhre 10 und dem Röntgendetektor 9 im Strahlengang der Röntgenröhre und in dem vom Detektor erfassten Bildbereich der Abbildungsbereich B, in dem das abzubildende Objekt O (z.B. Patient) angeordnet ist. Wie durch verschiedene Pfeile angedeutet (siehe auch Figur 3), ist das Aufnahmesystem, welches den C-Bogen 8, die Röntgenröhre 10 und den Röntgendetektor 9 umfasst, um zwei zueinander orthogonale Achsen, die C-Achse 19 und die P-Achse 20 (vgl. Figur 3) rotierbar. Die Rotation um die P-Achse erlaubt hierbei eine Rotation der Röhre 10 und des Detektors 9 aus der Bildebene heraus bzw. senkrecht zur Bildebene, die Drehung um die C-Achse 19 (die senkrecht zur Bildebene steht) erlaubt hierbei eine Rotation dieser Komponenten in der Bildebene.

Durch den C-Bogen 8 ist der Röntgendetektor 9 in festem Abstand zum und in fester Lage relativ zur Röntgenröhre 10 angeordnet. Der Abstand des und die relative Lage des Röntgendetektors 9 relativ zur Röntgenröhre 10 bleibt somit auch bei entsprechenden Rotationsbewegungen erhalten. Durch die Hubachse 21 und die Schubachse 22 (vgl. Figur 3) sind des weiteren translatorische Bewegungen des Aufnahmesystems 8 bis 10 in Richtung der P-Achse 20 und senkrecht zur P-Achse und zur C-Achse 19 möglich.

Fest mit dem C-Bogen 8 verbunden ist nun an diesem außenseitig ein Lagesensor 2 in Form eines Schwerkraftsensors angeordnet. Wie nachfolgend noch ausführlicher beschrieben wird, kann mit diesem Lagesensor 2 für jede momentane Lage des Systems Röntgenröhre-Röntgendetektor im Raum die Ausrichtung dieser Lage relativ zu der vordefinierten Referenzrichtung R bestimmt werden. Die vordefinierte Referenzrichtung R ist hier die Richtung der Erdbeschleunigung bzw. der Erdschwerevektor.

Im Bild gezeigt ist darüber hinaus die den eigentlichen C-Bogen tragende C-Bogen-Geräteeinheit 7. Diese ist zur Signalübertragung mit einer zentralen Recheneinheit 1 (welche beispielsweise einen PC umfassen kann) verbunden. Die vom Lagesensor 2 erfassten Daten bzw. momentanen Ausrichtungen der Röntgenröhre und des Röntgendetektors werden über Datenverbindungsleitungen an die zentrale Recheneinheit übertragen. Hier kann der Datenaustausch bidirektional ausgebildet sein, so dass von Seiten der zentralen Recheneinheit 1 die entsprechenden Sensorfunktionalitäten des Lagesensors 2 eingestellt bzw. verändert werden können.

Die zentrale Recheneinheit umfasst eine Speichereinheit 1a (hier: Festplatte), eine Recheneinheit 1b (hier: CPU und Hauptspeicher eines PCs) mit einer darin angeordneten Umrechnungseinheit 11 in Form einer Look-Up-Tabelle LUT sowie eine Rekonstruktionseinheit 1c (hier: separater Rekonstruktions-PC) und eine Hinweiseinheit 12, deren Funktion nachfolgend näher beschrieben wird. Die einzelnen Einheiten 1a, 1b, 1c und 12 sind untereinander zum Datenaustausch verbunden. Die einzelnen Einheiten können hierbei in Form von Hardware-Einheiten (z.B. Speicher oder Ähnliches) und/oder in Form von Software-Komponenten (Programme oder Datenstrukturen) realisiert sein.

Mit der zentralen Recheneinheit 1 ist eine Anzeigeeinheit 3 (Monitor oder Ähnliches) verbunden, mit der aufgenommene Röntgen-Projektionsbilder oder auch die rekonstruierten Schichtbilder angezeigt werden können.

Schließlich zeigt die Figur noch eine Kalibriereinheit 4 bis 6, welche im vorliegenden Fall ein Positionsmesssystem 6 in Form einer Positionskamera und einen Kalibrierkörper 4 mit Markern 5 umfasst. Diese Elemente 4 bis 6 sind lediglich bei der Kalibrierung der vorgestellten Anlage vorhanden und werden vor dem eigentlichen Aufnahmebetrieb bzw. Patientenbetrieb entfernt. Die Marker 5 sind die Marker, deren Position durch das Positionsmesssystem 6 erfasst wird. Dies können z.B. reflektierende Kugeln, LEDs o.Ä. sein.

Zu den Baugruppen des gezeigten Aufnahmesystems gehören somit ein Steuerrechner 1 mit eingebauter Videodigitalisierungskarte und Software, ein Lage- bzw. Beschleunigungssensor 2 und ein Anzeigesystem 3. Zur Kalibrierung wird der Kalibrierkörper 4 mit den Markern 5 für ein externes Positionsmesssystem 6 verwendet. Der Videoausgang des verwendeten C-Bogengerätes 7 wird mit der Videodigitalisierungskarte des Steuerrechners 1 verbunden. Der Lagesensor 2 wird wie beschrieben an der C-Bogeneinheit 8 befestigt, so dass eine starre Verbindung zwischen Lagesensor 2 und Röntgenbild-Empfänger 9 und Röntgenquelle 10 hergestellt ist. Der Datenausgang des Lagesensors 2 wird an den Steuerrechner 1 angeschlossen. Die Darstellung der Daten erfolgt dann auf dem Anzeigegerät 3. Zur Kalibrierung des Systems wird der Kalibrierkörper 4 am Röntgendetektor 9 angebracht und das Positionsmesssystem 6 am Steuerrechner 1 angeschlossen.

Wie bereits vorbeschrieben, erfolgt der Kalibrierungsbetrieb des gezeigten Röntgenbild-Aufnahmesystems wie folgt: Für eine Vielzahl unterschiedlicher Stellungen des Systems Röntgenröhre-Röntgendetektor 9, 10 im Raum wird mit Hilfe der Kalibriereinheit 4 bis 6 die Lage des Röntgendetektors 9 und der Röntgenröhre 10 im Raum erfasst. Hierzu wird der Kalibrierkörper 4 fest mit dem Röntgendetektor 9 verbunden. Bei dem Kalibrierkörper 4 handelt es sich um einen Körper festgelegter dreidimensionaler Geometrie, aus dessen Erfassung mit dem Kamerasystem 6 und paralleler Aufnahme und Auswertung eines Röntgenbildes eindeutig die relative Lage des Röhren-Detektorsystems 9, 10 im Raum bestimmt werden kann. Anhand der erfassten und ausgewerteten Röntgenbild-und Positionsdaten werden all diejenigen Positionsdaten hinsichtlich der Stellung des Systems 9, 10 im Raum bestimmt, ausgewertet und in der Speichereinheit 1a abgespeichert, die notwendig sind, um ein in dieser Stellung aufgenommenes Röntgen-Projektionsbild zur Rekonstruktion von Schichtbildern verwenden zu können.

Während der Kalibrierung werden die für die Rekonstruktion notwendigen Positionsdaten vollständig bestimmt. Durch die Durchführung der Kalibrierung an einer Vielzahl von unterschiedlichen Stellungen werden auch stellungsabhängige Einflüsse auf das Röntgensystem, z.B. das Verformen der Mechanik durch das hohe Eigengewicht, abgebildet.

Die Speicherung dieser notwendigen Positionsdaten erfolgt zusammen mit den zugehörigen Ausrichtungsdaten (die durch den Lagesensor 2 in derselben Stellung des Systems 9, 10 erfasst wurden) in der Speichereinheit la. Wurden Positionsdaten und zugehörige Ausrichtungen an einer ausreichenden Stützstellenanzahl bzw. an einer ausreichenden Zahl unterschiedlicher Stellungen des Systems 9, 10 im Raum erfasst und abgespeichert, so wird mit Hilfe der Recheneinheit 1b aus diesen Daten eine Look-Up-Tabelle LUT 11 generiert, die eine Umrechnung bzw. Transformation zwischen Ausrichtungsdaten und zugehörigen notwendigen Positionsdaten erlaubt. Die zusammen abgespeicherten Ausrichtungen und notwendigen Positionsdaten werden nachfolgend auch als Kalibrierdaten bezeichnet.

Bei Inbetriebnahme des Systems werden somit initial die Kalibrierdaten aufgenommen, die notwendig sind, um im späteren Untersuchungsbetrieb für jedes Röntgen-Projektionsbild die Lage des Bildes im Raum und die Position der Röntgenquelle im Raum (bzw. die Lage des Systems aus Röntgenröhre und Röntgendetektor 9, 10) bestimmen zu können. Dazu wird der Kalibrierkörper 4 am Bildverstärker angebracht und dessen Position kontinuierlich an einer ausreichenden Stützstellenzahl gemessen. Der Kalibrierkörper 4 besteht hier aus einer dreidimensionalen Geometrie, die auch im Röntgenbild sichtbar ist und zur Bestimmung der Abbildungseigenschaften des Röntgensystems mit Hilfe des Positionsmesssystems 6 dient. Immer dann, wenn die Aufnahme eines neuen Röntgen-Projektionsbildes festgestellt wird, bestimmt das Positionsmesssystem 6 die räumliche Lage des Kalibrierkörpers 4. Mit Hilfe der vorbestimmten Geometrie des Kalibrierkörpers 4, deren Abbildung im erfassten Röntgenbild und den durch das Messsystem erfassten Positionsdaten werden dann die Lage des Röntgen-Projektionsbildes bzw. die Position der Röntgenröhre 10 und des Detektors 9 und die Projektionseigenschaften des C-Bogens ermittelt und zusammen mit den Erdbeschleunigungswerten des Lagesensors 2 als Kalibrierdaten abgespeichert. Dieser Vorgang wird an ausreichend vielen Stützstellen bzw. Stellungen des Röhren-Detektorsystems 9, 10 im Raum wiederholt. In Abhängigkeit vom Systemzustand und den Sensordaten des Lagesensors 2 werden darüber hinaus, wie nachfolgend noch näher beschrieben, mit der Hinweiseinheit 12 Benutzerhinweise generiert, die den Anwender bei der Systemkalibrierung unterstützen bzw. ihm die notwendige Information darüber vermitteln, an welchen weiteren Stützstellen noch Kalibrierdaten zu erfassen sind.

Im eigentlichen Aufnahmebetrieb bzw. Patientenbetrieb werden nun die Elemente 4 bis 6, die lediglich für den vorbeschriebenen Kalibrierbetrieb notwendig sind, entfernt. Das Rechnersystem 1 bzw. dessen Speichereinheit 1a und Recheneinheit 1b sind nun so ausgebildet, dass nach Aufnahme eines Röntgen-Projektionsbildes (unter einer definierten Stellung des Röhren-Detektorsystems 9, 10 im Raum) anhand der dabei erfassten Sensorwerte des Lagesensors 2 (Ausrichtungsdaten relativ zur Referenzrichtung bzw. Erdschwererichtung R) diejenigen Positionsdaten des Röntgen-Projektionsbildes, die zu seiner Verwendung für die Bildrekonstruktion von Schichtbildern notwendig sind, aus den abgespeicherten Kalibrierdaten berechnet werden können. Hierzu wird die vorbeschriebene Look-Up-Tabelle herangezogen: Mit ihrer Hilfe wird die Ausrichtung relativ zur Referenzrichtung in die zugehörigen Positionsdaten transformiert. Dies kann beispielsweise mit Hilfe eines Spline-Interpolationsverfahrens geschehen, wie es dem Fachmann bekannt ist, mit dessen Hilfe aus den der Ausrichtung des aufgenommenen Röntgen-Projektionsbildes nächstliegenden Stützstellen-Ausrichtungen der Kalibrierdaten die notwendigen Positionsdaten des aufgenommenen Röntgen-Projektionsbildes bestimmt werden.

Wurden aus einer ausreichenden Anzahl von unterschiedlichen Raumrichtungen (beispielsweise über einen Umfang von 180° + Fächerwinkel des vom Detektor erfassten Röntgenstrahlfächers der Röntgenquelle) Röntgen-Projektionsbilder des Objektes O im Abbildungsbereich B aufgenommen, so können aus diesen aufgenommenen Bildern mit Hilfe der aus ihnen und den aus ihren Ausrichtungen mit Hilfe der LUT interpolierten notwendigen Positionsdaten mit Hilfe der Rekonstruktionseinheit 1c des Rechnersystems 1 die gewünschten Schichtbilder des Objektes O rekonstruiert werden.

Im Rahmen des Aufnahme- bzw. Patientenbetriebes wird die Hinweiseinheit 12 des Rechnersystems 1 dazu verwendet, festzustellen, aus welchen Raumrichtungen bzw. mit welchen Stellungen des Röhren-Detektorsystems 9, 10 für den gewählten Rekonstruktionsalgorithmus noch, zur Optimierung der Bildqualität der Rekonstruktionsbilder, weitere Röntgen-Projektionsbilder aufgenommen werden sollten. Die Hinweiseinheit 12 gibt dem Bediener dann durch eine Anzeige auf dem Monitor 3 entsprechende Anweisungen. Die Berechnung der weiteren notwendigen Projektionsrichtungen geschieht dabei auf Basis der berechneten Positionsdaten der bereits aufgenommenen Röntgen-Projektionsbilder. Somit überprüft während der Anwendung des Systems zur 3D-Bildaufnahme beim Patientenbetrieb die Software/Hardware des Steuerrechners den Videoeingang und erkennt anhand der Änderung des Bildinhalts die Aufnahme eines neuen Röntgen-Projektionsbildes. Erkennt der Steuerrechner 1 die Aufnahme eines solchen neuen Röntgen-Projektionsbildes, werden die Werte des Lagesensors 2 für diesen Zeitpunkt gespeichert. In den aufgenommenen Kalibrierdaten werden dann Positionsinformationen bzw. Positionsdaten mit ähnlichen Sensordaten (also mit ähnlicher Lage des Röntgendetektorsystems 9, 10) gesucht. Dies geschieht mit Hilfe geeigneter Interpolationsverfahren. Mit diesen Interpolationsverfahren werden die Lage des aufgenommenen Röntgen-Projektionsbildes und die Lage der aufnehmenden Röntgenquelle bestimmt. Die räumlich zugeordneten Projektionsbilder werden im System gespeichert. Schließlich wird eine 3D-Rekonstruktion mit Hilfe von dem Fachmann bekannten Rekonstruktionsalgorithmen aus den registrierten Projektionsbildern, also ein entsprechender 3D-Schichtbilddatensatz, berechnet. Die Projektionsbilder, der 3D-Schichtbilddatensatz und die räumlichen Zusammenhänge werden dem Benutzer angezeigt. In Abhängigkeit vom Systemzustand, den bereits aufgenommenen Röntgen-Projektionsbildern und den erfassten Sensordaten des Lagesensors 2 werden über die Einheit 12 Benutzerhinweise generiert, die den Anwender bei der Systembedienung, insbesondere bei der Ausrichtung des Aufnahmegerätes für noch aufzunehmende Projektionsrichtungen unterstützen.

Weitere Eigenschaften des im vorstehenden Ausführungsbeispiel beschriebenen erfindungsgemäßen Röntgenbild-Aufnahmesystems werden nun beschrieben.

### Abhängigkeit zwischen C-Bogenstellung und der Richtung der Erdbeschleunigung:

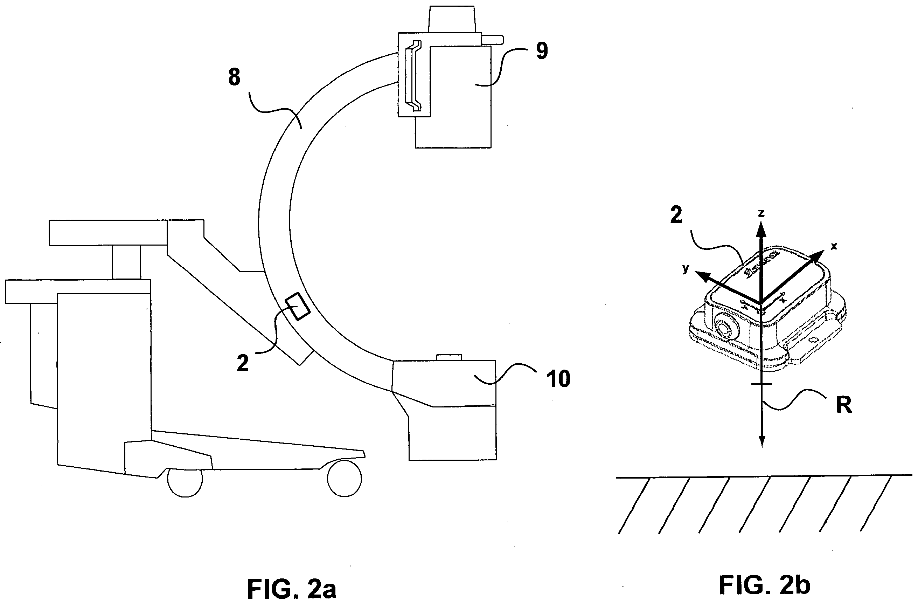

Eine konkrete Umsetzung der Erfindung besteht aus einem C-Bogen 8 und einem Beschleunigungssensor 2. Der Sensor ist fest mit der C-Struktur verbunden und damit unbeweglich gegenüber dem Bildverstärker und der Röntgenquelle (Fig. 2). Während der Benutzung wird mit Hilfe des Beschleunigungssensors die Richtung der Erdbeschleunigung gemessen. Der Messwert liegt in Form eines Vektors im internen Koordinatensystem des Sensors vor (siehe Fig. 2). Eine Änderung der Orientierung des Sensors verursacht aus Sicht des internen Koordinatensystems eine Änderung der Richtung des Vektors, solange die Rotationsachse nicht parallel zum Beschleunigungsvektor ist. Im Fall der Abbildung verursacht eine Drehung des Sensors um die Z-Achse also keine Änderung des Erdbeschleunigungsvektors.

Fig. 2a zeigt den C-Bogen mit montiertem Sensor 2. Fig. 2b zeigt das interne Koordinatensystem des Beschleunigungssensors 2 mit beispielhaftem Vektor für die Erdbeschleunigung. Eine Rotation des Sensors um die Achse des Erdbeschleunigungsvektors wirkt sich nicht auf die Richtung des Vektors im Bezugssystem aus.

Fig. 3 zeigt schematisch den Aufbau eines C-Bogens 8, inkl. der typischen Gelenke. An einer C-förmigen Struktur befinden sich die Röntgenquelle 10 und der Detektor 9 (Bildverstärker). Durch eine Drehung der C-Struktur um die C-Achse 19 oder P-Achse 20 können Röntgenbilder eines Objekts aus beliebigen Richtungen aufgenommen werden. Der Bildverstärker 9, die Röntgenquelle 10 und der Beschleunigungssensor 2 bewegen sich dabei auf einer konvexen Oberfläche (kann modellhaft als Kugel angenommen werden). Jede Bewegung der C-Struktur entspricht dabei einer Rotation des Sensors um die C- oder P-Achse. Solange diese Rotationsachse nicht parallel zum Erdbeschleunigungsvektor liegt, können die verschiedenen C-Bogenstellungen anhand der Erdbeschleunigungsrichtung eindeutig voneinander unterschieden werden.

Weitergehende Bewegungen der C-Strukturen sind durch das Benutzen der Hub- und Schubachse, sowie durch eine Bewegung des Fahrstativs möglich. Diese Bewegungen ändern nicht die Orientierung des Sensors im Raum und bewirken keine Änderung der Erdbeschleunigung im internen Koordinatensystem. Allerdings ist es theoretisch möglich, dass die Beschleunigungen, die während solcher Bewegungen auftreten, gemessen und zur Berechnung der Bewegungsbahn genutzt werden.

### Funktionsweise der Kalibrierung:

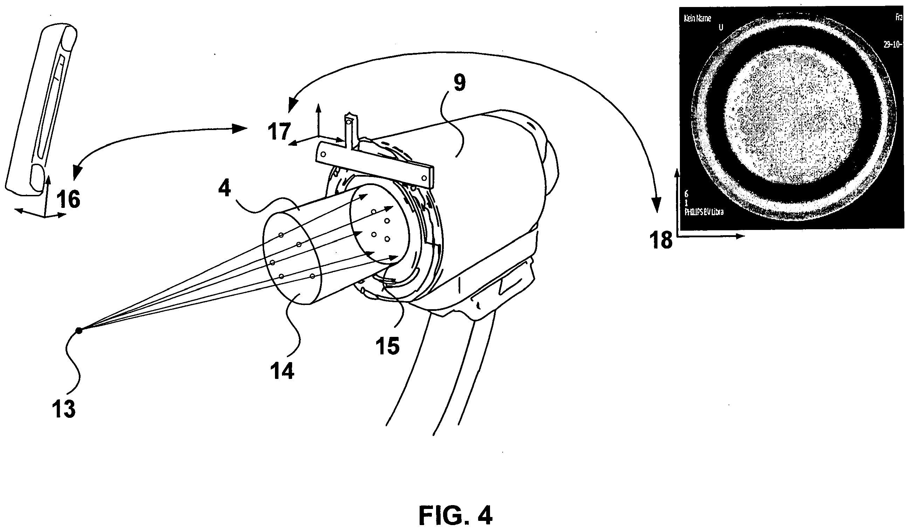

Ziel der Kalibrierung ist es, die Lage des Röntgenbildes und die Position relativ zu einem Basiskoordinatensystem BKS 16 zu bestimmen. Dieses BKS 16 wird im einfachsten Fall durch das optische Messsystem definiert, welches zur Kalibrierung benutzt wird. Fig. 4 zeigt den zweistufigen Kalibriervorgang für eine C-Bogenstellung. Die beiden Stufen werden im Folgenden beschrieben:

### 1. Bestimmung der Lage der Bildebene:

In einer Ebene (Registrierebene 15) nahe dem Bilderverstärker 9 sind Bleimarker an im Referenzkoordinatensystem definierten Positionen angebracht. Die Detektion der Markerschatten im Röntgenbild (Img)18 ermöglicht durch Point-to-Point-Matching die Bestimmung der Bildlage und Position relativ zum Referenzkoordinatensystem und folglich der Transformation *^{BKS}***T***_{Img}*. Dies ist möglich, da die Positionen der Bleimarker im Koordinatensystem CalBody 17 aus den Konstruktionsunterlagen bekannt sind und der Übergang zwischen CalBody 17 und BKS 16 durch das optische Messsystem gemessen wird.

### 2. Bestimmung der Position der Röntgenquelle:

In einer zweiten Ebene (Kalibrierebene 14) sind ebenfalls Bleimarker an bekannten Positionen angebracht. Die Markerschatten werden im Bild detektiert und mit Hilfe der aus Schritt 1 bekannten Transformation *^{BKS}***T***_{Img}* in 3D-Positionen umgerechnet. Dadurch können die Projektionsstrahlen für die Bleimarker der Kalibrierebene berechnet werden. Im Schnittpunkt dieser Strahlen befindet sich die Röntgenquelle 13.

Fig. 4 zeigt somit die Kalibrierung eines C-Bogens durch Ermittlung der Lage des Bildes und der Röntgenquelle 9 relativ zu einem Basiskoordinatensystem BKS 16. Ergebnis dieser Kalibrierung ist die Lage des Bildes im BKS, inkl. der Skalierungsparameter (Dimension der Bildpunkte). Dieser Kalibriervorgang wird für verschiedene Stellungen des C durchgeführt, so dass der gesamte Rotationsbereich abgedeckt ist. Für jede Stellung werden der aktuelle Erdbeschleunigungsvektor und die beiden Transformationen in einer Tabelle gespeichert.

### Herleitung der Positionsdaten aus den Schwerkraftdaten während der Benutzung:

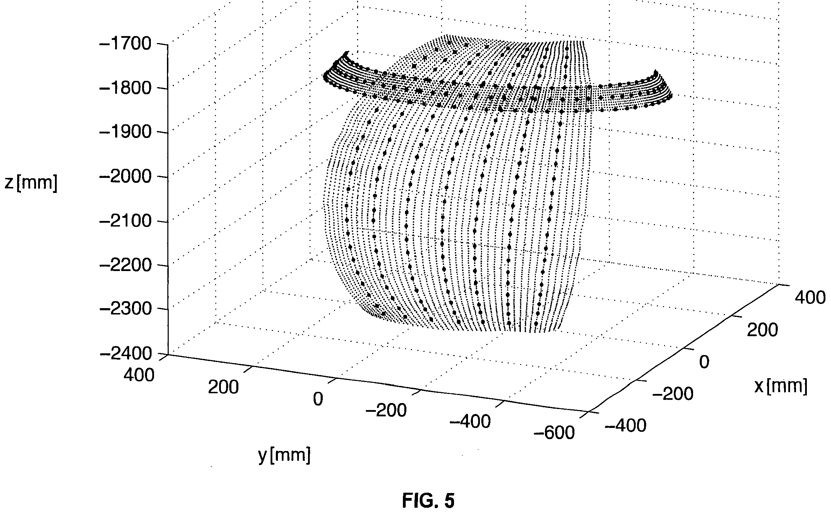

Während der eigentlichen Systembenutzung erkennt das System die Aufnahme eines neuen Röntgenbildes, z.B. durch die kontinuierliche Analyse des Videosignals. Liegt ein neues Röntgenbild vor, werden Beschleunigungsdaten eines definierten Zeitfensters zusammen mit den Bilddaten gespeichert. Durch Analyse der Streuung der Beschleunigungswerte während des Bildaufnahmezeitfensters kann kontrolliert werden, ob der C-Bogen während der Bildaufnahme in Ruhe war. Aus der Kalibriertabelle werden die Einträge geladen, die dem gemessenen Erdbeschleunigungsvektor am nahsten liegen. Durch Interpolation, z.B. mittels kubischen Splines, können die Positionsdaten für das aufgenommene Röntgenbild bestimmt werden. Fig. 5 zeigt eine 3D-Ansicht mit kalibrierten und interpolierten Stützstellen.

### Benutzungshinweise:

Bei der Benutzung des Systems muss der Anwender Röntgenbilder aus verschiedenen Richtungen aufnehmen, damit aus den Projektionsbildern Volumendaten rekonstruiert werden können. Dabei steigt die Rekonstruktionsqualität mit der Anzahl der Bilder und dem überstrichenen Winkelbereich. Um die Rekonstruktionsqualität zielgerichtet und effizient zu verbessern, ist es sinnvoll, mit der Hinweiseinheit 12 Benutzerhinweise zu generieren, die den Anwender bei der Ausrichtung des C-Bogens unterstützen. Anhand der Positionsdaten der bereits aufgenommenen Bilder kann berechnet werden, aus welcher Position weitere Bilder aufgenommen werden müssen, um die Rekonstruktionsqualität möglichst effektiv zu verbessern.

Solche Benutzerhinweise helfen ebenfalls bei der Ausrichtung des C-Bogens auf den Patienten.

### Funktionsbeschreibung:

Das erfindungsgemäße 3D-Bildgebungssystem erweitert Standard-C-Bögen um die 3D-Bildgebungsfunktionalität. Hierzu wird z.B. bei der Verwendung eines Bildverstärkers als Detektor ein Lagesensor an den C-Bogen angebracht und das Videobild vom Videoausgang abgegriffen. Der C-Bogen wird also weder baulich verändert, noch in seiner Funktionalität eingeschränkt. Einmalig muss das System mit Hilfe einer Positionskamera von einem Techniker kalibriert werden. Der Arzt kann wie gewohnt Bilder aufnehmen und diese betrachten. Zusätzlich steht ihm jederzeit ein aktuelles Rekonstruktionsergebnis zur Verfügung. Dieses kann vom Arzt in gewohnter Schichtansicht betrachtet werden. Um ein optimales Rekonstruktionsergebnis sicherzustellen, werden dem Arzt von der Hinweiseinheit 12 ideale Aufnahmepositionen empfohlen.

Durch die oben genannten Charakteristika ermöglicht das System eine kostengünstige und flexible 3D-Visualisierung für den prä-, intra- und postoperativen Einsatz.

Wichtige Bestandteile des 3D-CArm Bildgebungssystems sind dabei
1. Computer,
2. Bildschirm für die Visualisierung des rekonstruierten Volumens und der aufgenommen Röntgenbilder,
3. Lagesensor zur Bestimmung der C-Bogen-Ausrichtung im Raum,
4. Eingabegeräte, wie Maus und Tastatur,
5. Zubehör für die C-Bogen-Kalibrierung: Kalibrierkörper inkl. Tracker und Navigationskamera.

Die Funktion des Systems ist es, 3D-Bilddaten aus 2D-Röntgenbildern von Standard-C-Bögen zu erzeugen und diese darzustellen. Die 2D-Daten werden z.B. als Videosignal direkt vom C-Bogen abgegriffen, digitalisiert und analysiert. Die Funktionsweise des C-Bogens wird nicht eingeschränkt. Das System hat einen eigenen Spannungsversorgungsanschluss und wird darüber hinaus z.B. an dem analogen Videoausgang eines C-Bogens betrieben.

Nachdem das System mit dem C-Bogen verbunden und eingeschaltet wurde, startet die Applikation automatisch. Zunächst muss die gewünschte Aufnahmestrategie (Bildaufnahme entlang der Propellerachse bzw. P-Achse oder der C-Achse) ausgewählt werden. Die gewählte Aufnahmestrategie beeinflusst sowohl die C-Bogenstellungen, an denen Bilder aufgenommen werden müssen, als auch die Art des folgenden Dialogs zur Ausrichtung des C-Bogen. Zur Kontrolle der Ausrichtung wird das aktuelle Röntgenbild angezeigt. Der Benutzer muss in zwei verschieden Winkelstellung das Objekt in der Bildmitte positionieren. Als Hilfestellung wird in das Videobild ein Fadenkreuz eingeblendet, welches bei der Zentrierung des zu rekonstruierenden Objektes hilft. Anschließen wird die Mensch-Maschine-Schnittstelle mit dem Aufnahme-Assistent gestartet.

Der Aufnahme-Assistent 12 unterstützt den Benutzer bei der Aufnahme der Röntgenbilder. Es werden ihm die anzufahrenden C-Bogenstellungen mitgeteilt, an denen jeweils ein Bild gemacht werden muss. Die Rekonstruktion, die Bilderkennung und die Volumendarstellung arbeiten unabhängig voneinander, so dass auch während einer laufenden Rekonstruktion Röntgenbilder aufgenommen werden können.

Die Mensch-Maschine-Schnittstelle ermöglicht dem Benutzer jederzeit, das aktuelle Rekonstruktionsergebnis zu betrachten. Das Volumen wird in axialer, koronaler und sagittaler Schichtansicht visualisiert. Die registrierten Röntgenbilder werden in einem weiteren Fenster dargestellt. Mit dem Vorwärts- und Rückwärtsbutton können die Röntgenbilder durchgeschaut, beziehungsweise mit dem Modebutton in die Volumenansicht geschaltet werden. Alle Ansichten können gezoomt und einzeln auch in den Vollbildmodus geschaltet werden.

### Aufbau der Systemkomponenten:

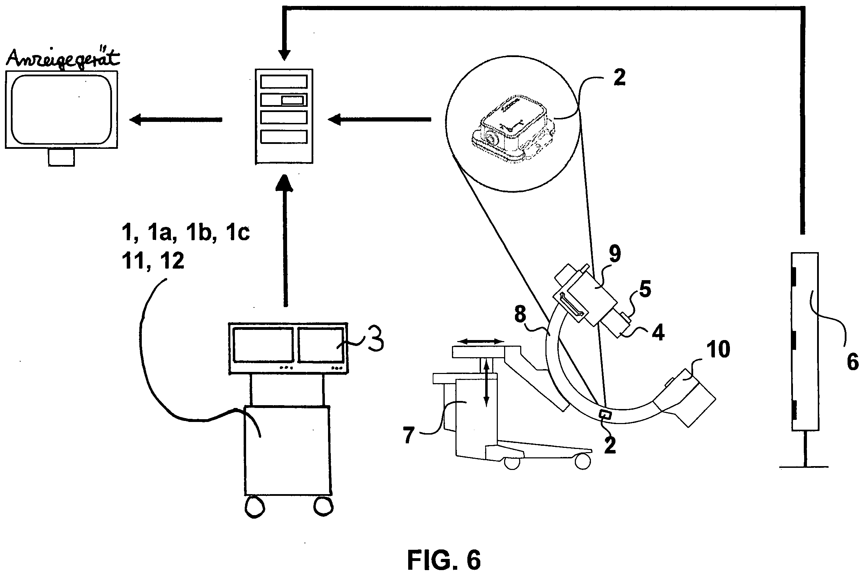

Zu den Baugruppen des Beispiel-Systems gehören ein PC 1 mit eingebauter Videodigitalisierungskarte, ein Beschleunigungssensor 2, ein Navigationssystem 6, ein Anzeigegerät 3 und ein Kalibrierkörper 4. Die Komponenten werden wie folgt elektrisch und mechanisch miteinander Verbunden (Fig. 6). Der Videoausgang (BNC) der Mobilen Betrachtungsstation wird mit der, in den PC eingebauten, Videodigitalisierungskarte verbunden. Der Beschleunigungssensor wird am C des C-Bogens befestigt (verschraubt oder festgeklebt) und mit dem Adapterkabel an den PC angeschlossen. Zur Visualisierung der Daten wird das mitgelieferte Anzeigegerät mit dem PC verbunden. Während der Kalibrierung des Systems sind folgende Komponenten an dem System angeschlossen. Der Kalibrierkörper wird am Bildverstärker angebracht (verschrauben oder verkleben), wobei der Tracker zur offenen Seite des C zeigen muss. Das Navigationssystem wird über ein serielles Kabel ebenfalls an den PC angeschlossen und frontseitig zum C-Bogen positioniert.

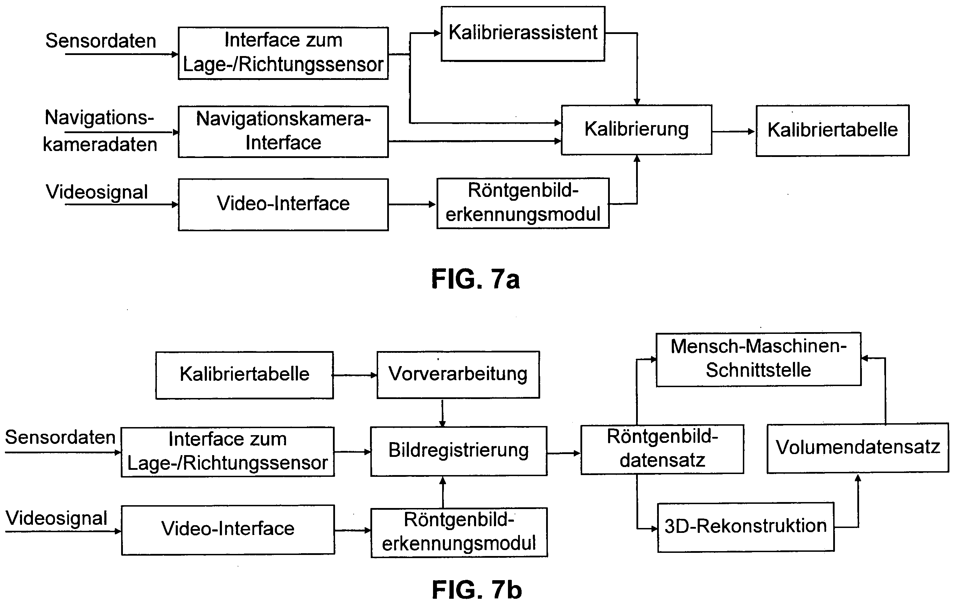

### Dynamisches Verhalten der Software während der Kalibrierung (Fig. 7a):

Nachdem die Software im Kalibriermodus gestartet wurde, wird das System auf die Funktionsfähigkeit der zur Kalibrierung notwendigen Komponenten überprüft. Nach dem Bestimmen der Sensorlage relativ zum C-Bogen (mittels zweier definierter C-Bogenstellungen) wird das Röntgenbilderkennungsmodul aktiviert und das digitalisierte Videobild auf neue Röntgenbilder überprüft.

Der Benutzer fährt mit dem C-Bogen die vom Kalibrierassistenten angezeigten Positionen an, tätigt an diesen Stellen je eine Röntgenaufnahme und wartet jeweils auf eine positive Rückmeldung des Systems.

Sobald ein neues Röntgenbild erkannt wird und dieses über eine gewisse Zeit stabil am Ausgang anliegt, wird das Bild der Kalibrierung zugeführt. Die Kalibrierung erkennt die Marker im inneren Bildbereich und berechnet daraus die Lage der Bildebene relativ zum BV-Tracker. Mit Hilfe der äußeren Marker und deren Projektionen im Bild wird die Position der Röntgenquelle relativ zur Bildmitte bestimmt. Um Bildstörungen, die bei der Digitalisierung entstehen, zu unterdrücken, können 19 zusätzliche Videobilder aufgenommen und einzeln kalibriert werden. Von den 20 bestimmten Bildparametern wird der Median errechnet. Die ermittelten Parameter und die Positions- und Lagedaten des Kalibrierkörpers werden jeweils mit den aktuellen Lagedaten je in einer Kalibriertabelle abgespeichert.

### Beschreibung der Softwarekomponenten während der Kalibrierung des C-Bogens:

| | |
|---|---|
| **Navigationskamera-Interface:** | Stellt dem System die Position und die Ausrichtung des Kalibrierkörpers im Navigationskamerasystem zur Verfügung. Die Daten werden über 100 Werte gemittelt, um das Rauschen zu unterdrücken. Zusätzlich findet eine Bewegungsüberwachung statt. |
| **Beschleunigungssensor-Interface:** | Kommunikation mit dem Beschleunigungssensor. Außerdem werden die Beschleunigungswerte in X-, Y- und Z-Richtung gepuffert und können über einen beliebigen Zeitraum (maximal Pufferlänge) gemittelt abgefragt werden. Eine Analysefunktion ermöglicht eine Störungsdetektion über den angeforderten Mittlungszeitraum. |
| **Video-Interface:** | Interface zur Videodigitalisierungskarte. Sie stellt dem System das aktuelle Videobild zur Verfügung. |
| **Röntgenbilderkennungsmodul:** | Untersucht zyklisch das Videobild mit Hilfe eines Differenzbildverfahrens auf Unterschiede, um so neue Röntgenbilder zu erkennen. Überwacht werden nur bestimmte Bereiche unter Berücksichtigung der Zeit- und Bildeigenschaften des C-Bogens. Beim Überschreiten eines Schwellwertes wird das aktuelle Videobild dem Bildkalibrierungsmodul als neues Bild zugeführt. |
| **Kalibrierassistent:** | Die Mensch-Maschine-Schnittstelle zeigt die nächste anzufahrende Position und die zurückzulegende Winkeldifferenz an. |
| **Kalibrierung:** | Erzeugt aus den Sensor-, Navigations- und, aus dem Röntgenbild gewonnen, Geometriedaten den aktuellen Kalibrierdatensatz. Dieser besteht aus der Lage des Kalibrierkörpers im Raum, der Bildlage relativ zum Kalibrierköper sowie der relativen Lage der Röntgenquelle. |
| **Kalibriertabelle:** | Die Lage des Kalibrierkörpers im Raum, die Bildlage relativ zum Kalibrierköper sowie die relative Lage der Röntgenquelle werden in getrennten Dateien abgelegt. |

### Dynamisches Verhalten der Software während des Betriebs (Fig. 7b):

Nach dem Programmstart teilt der Benutzer dem System mit, welche Aufnahmestrategie er benutzen möchte. Hierzu wird ein Aufnahmestrategieauswahldialog angezeigt, welcher nach Auswahl die entsprechenden Kalibriertabellen lädt und anschließend spezifische C-Bogenausrichtungsanweisungen an den Benutzer ausgibt. Um den Nutzer zu unterstützen, wird das aktuelle Videobild ausgegeben.

Die geladenen Kalibriertabellen durchlaufen zunächst eine Vorverarbeitung. Hierbei werden neue Stützstellen extrapoliert und zwischen allen Stützstellen neue Werte interpoliert. Anschließend wird das Röntgenbilderkennungsmodul aktiviert.

Die Benutzerführung stellt visuell die nächste anzufahrende C-Bogenstellung dar. Die Röntgenbilderkennung kontrolliert periodisch das digitalisierte Videosignal vom analogen Videoausgang des C-Bogens. Sobald ein neues Röntgenbild erkannt wird und dieses über eine gewisse Zeit stabil am Ausgang anliegt, wird es als neues Röntgenbild ins System übernommen und zusammen mit den gemittelten Lagedaten der Bildregistrierung zugeführt. Diese umfasst eine Helligkeitskorrektur sowie die Maskierung und Invertierung des Bildes. Anhand der Lagedaten des Sensors werden nahe gelegene Stützstellen gesucht und zwischen diesen linear Interpoliert. Die so gewonnenen Positions-daten werden dem Bild zugewiesen und gespeichert. Anschließend wird das Bild als neues Röntgenbild in der Mensch-Maschine-Schnittstelle angezeigt und in der Röntgenbildrekonstruktionsliste hinzugefügt. Das System springt nun wieder in den Videoüberwachungsmodus und ist bereit für neue Röntgenbilder.

Der Rekonstruktionsalgorithmus stellt fest, ob neue Röntgenbilder vorliegen und startet gegebenenfalls eine neue Rekonstruktion über alle Bilder. Der aktuelle Fortschritt wird in einem Fortschrittsbalken angezeigt. Bei durchgeführter Rekonstruktion wird das neue Volumen in die Mensch-Maschine-Schnittstelle geladen und der Kontrast automatisch geregelt. Der 3D-Rekonstruktionsalgorithmus arbeitet unabhängig von der Röntgenbilderkennung und der Bildregistrierung so, dass das System neue Röntgenbilder aufnehmen kann, während die aktuelle Rekonstruktion noch nicht abgeschlossen ist. Außerdem können parallel das Ergebnis der letzten Rekonstruktion und alle registrierten Röntgenbilder mit der Mensch-Maschine-Schnittstelle betrachtet werden.

Die Mensch-Maschine-Schnittstelle ermöglicht dem Benutzer jederzeit das aktuelle Rekonstruktionsergebnis zu betrachten. Das Volumen wird in axialer, koronaler und sagittaler Schichtansicht visualisiert. Diese können gezoomt und einzeln auch in den Vollbildmodus geschaltet werden. Die registrierten Röntgenbilder werden in einem weiteren Fenster dargestellt. Mit dem Vorwärts- und Rückwärtsbutton können die Röntgenbilder durchgeschaut, beziehungsweise mit dem Modebutton in die Volumenansicht geschaltet werden. Es steht auch für dieses Fenster der Vollbildmodus zur Verfügung.

### Beschreibung der Softwarekomponenten während des Rekonstruktionsbetriebs (Fig. 7b):

| | |
|---|---|
| **Video-Interface:** | Interface zur Videodigitalisierungskarte. Sie stellt dem System das aktuelle Videobild zur Verfügung. |
| **Beschleunigungssensor-Interface:** | Kommunikation mit dem Beschleunigungssensor. Außerdem werden die Beschleunigungswerte in X-, Y- und Z-Richtung gepuffert und können über einen beliebigen Zeitraum (maximal Pufferlänge) gemittelt abgefragt werden. Eine Analysefunktion ermöglicht eine Bewegungsdetektion über den angeforderten Mittlungszeitraum. |
| **Röntgenbilderkennungsmodul:** | Untersucht zyklisch das Videobild mit Hilfe eines Differenzbildverfahrens auf Unterschiede, um so neue Röntgenbilder zu erkennen. Überwacht werden nur bestimmte Bereiche unter Berücksichtigung der Zeit- und Bildeigenschaften des C-Bogens. Beim Überschreiten eines Schwellwertes wird das aktuelle Videobild als neues Bild dem Bildregistrierungsmodul zugeführt. |
| **Kalibriertabelle (LUT):** | Enthält die Zuordnungstabellen der Abbildungseigenschaften zu den Werten des Beschleunigungssensors. |
| **Vorverarbeitung:** | Extrapoliert zusätzliche Stützstellen aus den geladenen Kalibriertabellen und Interpoliert Stützstellen im 1° Abstand. |
| **Bildregistrierung:** | Unterzieht das Röntgenbild einer Vorverarbeitung und anhand der aktuellen Beschleunigungswerte werden die nächstgelegen Stützstellen bestimmt und zwischen ihnen linear die entsprechenden Bildabbildungsparameter interpoliert und dem Bild zugewiesen. |
| **Röntgenbilddatensatz:** | Liste aller bisher aufgenommen Röntgenbilder |
| **3D**-**Rekonstruktion:** | Startet eine neue 3D-Volumenrekonstruktion, wenn neue Bilder vorliegen und die vorherige Rekonstruktion abgeschlossen wurde. Des Weiteren werden die Kontrastparameter des Volumens für die MMS bestimmt. |
| **Volumendatensatz:** | Enthält das aktuell fertig rekonstruierte Volumen |
| **Mensch-Maschinen-Schnittstellen:** | Stellt den aktuellen Volumendatensatz in Schichtansicht, sowie die bisher aufgenommen Röntgenbilder bzw. 3D-Ansichten der Schichten dar. |

### Erläuterung zu den Figuren:

- 1: zentrale Recheneinheit mit:
- 1a: Speichereinheit
- 1b: Recheneinheit
- 1c: Rekonstruktionseinheit
- 2: Lagesensor
- 3: Anzeigeeinheit
- 4: Kalibrierkörper
- 5: Marker
- 6: Positionsmesssystem
- 7: C-Bogengerät
- 8: C-Bogeneinheit
- 9: Röntgenbilddetektor
- 10: Röntgenröhre
- 11: Umrechnungseinheit
- 12: Hinweiseinheit
- 13: Röntgenquellenposition
- 14: Kalibrierebene des Kalibrierkörpers
- 15: Registrierebene des Kalibrierkörpers
- 16: Basiskoordinatensystem BKS
- 17: Kalibrierkörperkoordinatensystem CalBody
- 18: Bildkoordinatensystem img
- 19: C-Achse
- 20: P-Achse
- 21: Hubachse
- 22: Schubachse

- B: Abbildungsbereich
- O: abzubildendes Objekt
- R: Referenzrichtung

## Patentansprüche

1. Röntgenbild-Aufnahmesystem zur Aufnahme von Röntgenprojektionsbildern und von Ausrichtungsinformationen für aufgenommene Röntgenprojektionsbilder, umfassend
eine Röntgenröhre (10) und einen im Strahlengang der Röntgenröhre (10) angeordneten Röntgenbilddetektor (9) zur Aufnahme von Röntgenprojektionsbildern eines zwischen der Röntgenröhre (10) und dem Röntgendetektor (9) in einem Abbildungsbereich (B) ortsfest anordenbaren und/oder angeordneten, abzubildenden Objektes (O), wobei die Röntgenröhre (10) und der Röntgendetektor (9) relativ zueinander ortsfest angeordnet und zumindest abschnittsweise um den Abbildungsbereich (B) herum bewegbar sind,
einen relativ zur Röntgenröhre (10) und zum Röntgendetektor (9) ortsfest angeordneten Lagesensor (2), mit dem im Moment der Aufnahme eines Röntgenprojektionsbildes die momentane Ausrichtung der Röntgenröhre (10) und des Röntgendetektors (9) relativ zu einer vordefinierten Referenzrichtung (R) bestimmbar ist, und
eine Speichereinheit (1a) zum Abspeichern aufgenommener Röntgenprojektionsbilder samt der jeweils zugehörigen momentanen Ausrichtung der Röntgenröhre (10) und des Röntgendetektors (9),
eine mit einer Speichereinheit (1a) zum Datenaustausch verbundene Recheneinheit (1b),
eine mit der Speichereinheit (1a) und/oder der Recheneinheit (1b) zum Datenaustausch verbundene Rekonstruktionseinheit (1c) zur Erstellung einer Schichtbildrekonstruktion aus den abgespeicherten Röntgenprojektionsbildern und den zugehörigen Positionsdaten,
wobei die Recheneinheit (1b) für jedes zur Rekonstruktion herangezogene Röntgenprojektionsbild diejenigen Positionsdaten des Röntgenprojektionsbildes, die für die Berechnung der Schichtbilder notwendig sind, aus den abgespeicherten, zugehörigen momentanen Ausrichtungen berechnet,
wobei die Speichereinheit (1a) und / oder die Recheneinheit (1b) eine vorbestimmte Umrechnungseinheit (11) umfasst, zur Umrechnung einer Ausrichtung relativ zur Referenzrichtung (R) in die zu dieser Ausrichtung gehörenden, zur Rekonstruktion der Schichtbilder notwendigen Positionsdaten.

2. Röntgenbild-Aufnahmesystem nach dem vorhergehenden Anspruch,
***dadurch gekennzeichnet, dass***
der Lagesensor (2) ein Beschleunigungssensor, insbesondere ein Schwerkraftsensor, ist, mit dem die momentane Ausrichtung der Röntgenröhre (10) und des Röntgendetektors (9) relativ zu einer vordefinierten Beschleunigungsrichtung, insbesondere der Richtung der Erdbeschleunigung, als Referenzrichtung (R) bestimmbar ist.

3. Röntgenbild-Aufnahmesystem nach einem der vorhergehenden Ansprüche,
***dadurch gekennzeichnet, dass***
die Umrechnungseinheit (11) mittels einer Kalibriereinheit (4, 5, 6) vorbestimmbar ist und/oder vorbestimmt ist,
wobei bevorzugt die Kalibriereinheit (4, 5, 6) einen in dem vom Röntgendetektor (9) erfassbaren und/oder erfassten Strahlengang der Röntgenröhre (10) und relativ zur Röntgenröhre (10), zum Röntgendetektor (9) oder zur Röntgenröhre (10) und zum Röntgendetektor (9) ortsfest angeordneten Kalibrierkörper (4, 5) aufweist.

4. Röntgenbild-Aufnahmesystem nach dem vorhergehenden Anspruch,
***dadurch gekennzeichnet, dass***
die Kalibriereinheit (4, 5, 6) ein Positionsmesssystem (6), insbesondere eine Positionskamera und/oder eine Navigationssystem-Kamera, aufweist,
mit dem die räumliche Lage und/oder Orientierung des Kalibrierkörpers (4, 5) zur Bestimmung von Positionsdaten, die für die Berechnung der Schichtbilder einer Schichtbildrekonstruktion notwendig sind, abtastbar ist und
mit dem diese aus einer Abtastung des Kalibrierkörpers (4, 5) in einer definierten räumlichen Lage und/oder Orientierung gewonnenen Positionsdaten zusammen mit der mittels des Lagesensors (2) im Moment dieser Abtastung bestimmten momentanen Ausrichtung der Röntgenröhre (10) und des Röntgendetektors (9) relativ zu der vordefinierten Referenzrichtung (R) als Kalibrierdaten abspeicherbar sind und/oder zur Abspeicherung, bevorzugt zur Abspeicherung in der Speichereinheit (la), übermittelbar sind.

5. Röntgenbild-Aufnahmesystem nach dem vorhergehenden Anspruch,
***dadurch gekennzeichnet, dass***
die für die Berechnung der Schichtbilder der Rekonstruktion notwendigen Positionsdaten mit der Recheneinheit (1b) unter Verwendung der abgespeicherten, zugehörigen momentanen Ausrichtungen der für die Rekonstruktion zu verwendenden Röntgenprojektionsbilder aus den Kalibrierdaten mit Hilfe eines Interpolationsverfahrens, insbesondere mittels einer Spline-basierten Interpolation, gewinnbar sind.

6. Röntgenbild-Aufnahmesystem nach einem der vorhergehenden Ansprüche
***gekennzeichnet durch***
genau eine vordefinierte Referenzrichtung (R).

7. Röntgenbild-Aufnahmesystem nach einem der vorhergehenden Ansprüche
***gekennzeichnet durch***
eine Hinweiseinheit (12), mit der auf Basis der zugehörigen momentanen Ausrichtungen bereits aufgenommener Röntgenprojektionsbilder weitere Ausrichtungen der Röntgenröhre (10) und des Röntgendetektors (9) berechenbar und angebbar sind, unter denen weitere Röntgenprojektionsbilder zur Verwendung für eine nachfolgende Schichtbildrekonstruktion aufzunehmen sind.

8. Röntgenbild-Aufnahmesystem nach dem vorhergehenden Anspruch,
***dadurch gekennzeichnet, dass***
die weiteren Ausrichtungen aus berechneten Positionsdaten bereits aufgenommener Röntgenprojektionsbilder berechenbar sind.

9. Röntgenbild-Aufnahmesystem nach einem der vorhergehenden Ansprüche
***gekennzeichnet durch***
eine Anzeigeeinheit (3) zur Anzeige von aufgenommenen Röntgenprojektionsbildern und/oder von Schichtbildern einer auf Basis von aufgenommenen Röntgenprojektionsbildern durchgeführten Schichtbildrekonstruktion, und/oder **durch** eine C-Bogen-Einheit, insbesondere eine C-Bogen-Einheit, deren C-Bogen (8) um zwei bevorzugt orthogonal zueinander ausgerichtete Achsen (C-Achse und P-Achse) rotierbar ist, an deren einem C-Bogen-Ende die Röntgenröhre (10) und an deren anderem C-Bogen-Ende der Röntgendetektor (9) fixiert ist.

10. Röntgenbild-Aufnahmesystem nach dem vorhergehenden Anspruch,
***dadurch gekennzeichnet, dass***
der Lagesensor (2) so am C-Bogen (8) fixiert ist, dass eine starre Verbindung zwischen dem Lagesensor (2), der Röntgenröhre (10) und dem Röntgendetektor (9) ausgebildet ist.

11. Röntgenbild-Aufnahmeverfahren zur Aufnahme von Röntgenprojektionsbildern und von Ausrichtungsinformationen für aufgenommene Röntgenprojektionsbilder, wobei
eine Röntgenröhre (10) und ein im Strahlengang der Röntgenröhre (10) platzierter Röntgenbilddetektor (9) zur Aufnahme von Röntgenprojektionsbildern eines abzubildenden Objektes (O), das zwischen der Röntgenröhre (10) und dem Röntgendetektor (9) in einem Abbildungsbereich (B) ortsfest angeordnet wurde, die relativ zueinander ortsfest angeordnet sind, zumindest abschnittsweise um den Abbildungsbereich (B) herum bewegt werden,
wobei ein Lagesensor (2) relativ zur Röntgenröhre (10) und zum Röntgendetektor (9) ortsfest angeordnet wird,
wobei mit dem Lagesensor (2) im Moment der Aufnahme eines Röntgenprojektionsbildes die momentane Ausrichtung der Röntgenröhre (10) und des Röntgendetektors (9) relativ zu einer vordefinierten Referenzrichtung (R) bestimmt wird, wobei die aufgenommenen Röntgenprojektionsbilder samt der jeweils zugehörigen momentanen Ausrichtung der Röntgenröhre (10) und des Röntgendetektors (9) abgespeichert werden, und
wobei zum Zweck einer auf mehreren aufgenommenen Röntgenprojektionsbildern basierenden Schichtbildrekonstruktion für zu dieser Rekonstruktion herangezogene aufgenommene Röntgenprojektionsbilder diejenigen Positionsdaten dieser Röntgen-projektionsbilder, die für die Berechnung der Schichtbilder der Rekonstruktion notwendig sind, aus den abgespeicherten, zugehörigen momentanen Ausrichtungen berechnet werden, wobei eine Umrechnung einer Ausrichtung relativ zur Referenzrichtung (R) in die zu dieser Ausrichtung gehörenden, zur Berechnung der Schichtbilder der Rekonstruktion notwendigen Positionsdaten erfolgt.

12. Röntgenbild-Aufnahmeverfahren nach dem vorhergehenden Anspruch,
***dadurch gekennzeichnet, dass***
nach Anbringen des Lagesensors (2) gemäß Anspruch 11 ein Röntgenbild-Aufnahmesystem nach einem der Ansprüche 2 bis 10 zur Aufnahme eingesetzt wird.

## Claims

1. X-ray image recording system for recording X-ray projection images and orientation information for recorded X-ray projection images, comprising
an X-ray tube (10) and an X-ray image detector (9) disposed in the optical path of the X-ray tube (10) for recording X-ray projection images of an object (O) to be imaged, which can be disposed and/or is disposed in a fixed manner between the X-ray tube and the X-ray detector in an imaging region (B), the X-ray tube (10) and the X-ray detector (9) being disposed in a fixed manner relative to each other and being moveable around the imaging region (B) at least in a sector,
a position sensor (2) which is disposed in a fixed manner relative to the X-ray tube (10) and to the X-ray detector (9), and with which, at the moment of recording of an X-ray projection image, the momentary orientation of the X-ray tube (10) and of the X-ray detector (9) relative to a pre-defined reference direction (R) can be determined, and
a memory unit (1a) for storing recorded X-ray projection images together with the respectively associated momentary orientation of the X-ray tube (10) and of the X-ray detector (9),
a processing unit (1b) which is connected to the memory unit (1a) for data exchange,
a reconstruction unit (1c) which is connected to the memory unit (1a) and/or to the processing unit (1b) for data exchange, and with which, from the stored X-ray projection images and the associated position data, a tomographic image reconstruction can be performed,
wherein the processing unit (1b) calculates, for each X-ray projection image used for the reconstruction, those position data of the X-ray projection image, which are required for the calculation of the tomographic images, from the stored, associated momentary orientations,
wherein the memory unit (1a) and/or the processing unit (1b) includes a predetermined conversion unit (11) for converting an orientation relative to the reference direction (R) into the position data associated with this orientation and required for the reconstruction of the tomographic images.

2. X-ray image recording system according to the preceding claim,
***characterised in that***
the position sensor (2) is an acceleration sensor, in particular a gravity sensor, with which the momentary orientation of the X-ray tube (10) and of the X-ray detector (9) can be determined relative to a predefined acceleration direction as the reference direction (R), in particular relative to the direction of gravitational acceleration.

3. X-ray image recording system according to one of the preceding claims,
***characterised in that***
the conversion unit (11) can be preset and/or is preset by means of a calibration unit (4, 5, 6),
wherein preferably the calibration unit (4, 5, 6) has a calibration body (4, 5), which is disposed in the optical path of the X-ray tube (10) which can be detected and/or is detected by the X-ray detector (9) and which is disposed in a fixed manner relative to the X-ray tube (10), to the X-ray detector (9) or to the X-ray tube (10) and to the X-ray detector (9).

4. X-ray image recording system according to the preceding claim,
***characterised in that***
the calibration unit (4, 5, 6) has a position measuring system (6), in particular a position camera and/or a navigation system camera,
with which the spatial position and/or orientation of the calibration body (4, 5) can be scanned for determining position data which are required for the calculation of the tomographic images of a tomographic image reconstruction, and
with which these position data, which are obtained from scanning the calibration body (4, 5) in a defined spatial position and/or orientation, together with the momentary orientation of the X-ray tube (10) and of the X-ray detector (9) relative to the predefined reference direction (R), which is determined by means of the position sensor (2) at the moment of this scanning, can be stored as calibration data and/or can be transmitted for storage, preferably for storage in the memory unit (1a).

5. X-ray image recording system according to the preceding claim,
***characterised in that***
the position data required for calculation of the tomographic images of the reconstruction can be obtained, with the processing unit (1b), from the calibration data with the help of an interpolation method, in particular by means of a spline-based interpolation, by using the stored, associated momentary orientations of the X-ray projection images to be used for the reconstruction.

6. X-ray image recording system according to one of the preceding claims,
***characterised by***
precisely one predefined reference direction (R).

7. X-ray image recording system according to one of the preceding claims,
***characterised by***
an instruction unit (12) with which, on the basis of the associated momentary orientations of already recorded X-ray projection images, further orientations of the X-ray tube (10) and of the X-ray detector (9) can be calculated and indicated, at which further X-ray projection images for use for a subsequent tomographic image reconstruction should be recorded.

8. X-ray image recording system according to the preceding claim,
***characterised in that***
the further orientations can be calculated from calculated position data of already recorded X-ray projection images.

9. X-ray image recording system according to one of the preceding claims,
***characterised by***
a display unit (3) for displaying recorded X-ray projection images and/or tomographic images of a tomographic image reconstruction performed on the basis of recorded X-ray projection images and/or by a C-arm unit, in particular a C-arm unit the C-arm (8) of which can be rotated about two axes (C-axis and P-axis) which are orientated preferably orthogonally relative to each other, on the one C-arm end of which the X-ray tube (10) and on the other C-arm end of which the X-ray detector (9) is fixed.

10. X-ray image recording system according to the preceding claim,
***characterised in that***
the position sensor (2) is fixed on the C-arm (8) such that a rigid connection between the position sensor (2), the X-ray tube (10) and the X-ray detector (9) is formed.

11. X-ray image recording method for recording X-ray projection images and orientation information for recorded X-ray projection images,
wherein an X-ray tube (10) and an X-ray image detector (9), the latter being placed in the optical path of the X-ray tube (10) and being for recording X-ray projection images of an object (O) to be imaged, which object has been disposed in a fixed manner between the X-ray tube (10) and the X-ray detector (9) in an imaging region (B), which (9, 10) are disposed in a fixed manner relative to each other, are moved around the imaging region (B) at least in a sector,
wherein a position sensor (2) is disposed in a fixed manner relative to the X-ray tube (10) and to the X-ray detector (9),
wherein the momentary orientation of the X-ray tube (10) and of the X-ray detector (9) relative to a predefined reference direction (R) is determined with the position sensor (2) at the moment of the recording of an X-ray projection image,
wherein the recorded X-ray projection images are stored together with the respectively associated momentary orientation of the X-ray tube (10) and of the X-ray detector (9), and
wherein, for the purpose of a tomographic image reconstruction which is based on a plurality of recorded X-ray projection images and for recorded X-ray projection images used for this reconstruction, those position data of these X-ray projection images, which are required for calculation of the tomographic images of the reconstruction, are calculated from the stored, associated momentary orientations, wherein a conversion of an orientation relative to the reference direction (R) into the position data associated with this orientation and required for the calculation of the tomographic images of the reconstruction is done.

12. X-ray image recording method according to the preceding claim,
***characterised in that***
after arranging the position sensor (2) according to claim 11, an X-ray image recording system according to one of the claims 2 to 10 is used for the recording.

## Revendications

1. Système de capture d'images à rayons X pour la capture d'images de projection aux rayons X et d'informations d'orientation pour des images de projection à rayons X, comprenant
un tube à rayons X (10) et un détecteur d'image à rayons X (9) disposé dans le trajet des faisceaux du tube à rayons X (10), pour la capture d'images de projection à rayons X d'un objet (O) à représenter, disposé et/ou pouvant être disposé de manière stationnaire dans une zone de représentation (B) entre le tube à rayons X (10) et le détecteur de rayons X (9), le tube à rayons X (10) et le détecteur de rayons X (9) étant disposés de manière stationnaire l'un par rapport à l'autre et étant mobiles au moins partiellement autour de la zone de représentation (B),
un capteur de position (2) disposé de manière stationnaire par rapport au tube à rayons X (10) et au détecteur de rayons X (9), avec lequel, au moment de la capture d'une image de projection à rayons X, l'orientation momentanée du tube à rayons X (10) et du détecteur de rayons X (9) par rapport à une direction de référence (R) prédéfinie peut être déterminée et
une unité de mémoire (1a) pour l'enregistrement d'images de projection à rayons X avec l'orientation momentanée correspondante du tube à rayons X (10) et du détecteur de rayons X (9),
une unité de calcul (1b) connectée avec une unité de mémoire (1a) pour l'échange de données, une unité de reconstitution (1c), connectée avec l'unité de mémoire (1a) et/ou l'unité de calcul (1b) pour l'échange de données, pour la création d'une reconstitution d'image superposée à partir des images de projection à rayons X et des données de position correspondantes,
l'unité de calcul (1b) calculant, pour chaque image de projection à rayons X utilisée pour la reconstitution, les données de position des images de projection à rayons X qui sont nécessaires pour le calcul des images superposées, à partir des orientations momentanées enregistrées correspondantes,
l'unité de mémoire (1a) et/ou l'unité de calcul (1b) comprenant une unité de conversion (11) prédéterminée, pour la conversion d'une orientation par rapport à la direction de référence (R) dans les données de reconstitution correspondant à cette orientation et nécessaires pour la reconstitution des images superposées.

2. Système de capture d'image à rayons X selon la revendication précédente, **caractérisé en ce que** le capteur de position (2) est un capteur d'accélération, plus particulièrement un capteur de gravité, avec lequel l'orientation momentanée du tube à rayons X (10) et du détecteur de rayons X (9) par rapport à une direction d'accélération prédéfinie, plus particulièrement la direction de l'accélération terrestre, peut être déterminée en tant que direction de référence (R).

3. Système de capture d'image à rayons X selon l'une des revendications précédentes, **caractérisé en ce que** l'unité de conversion (11) peut être prédéterminée et/ou est prédéterminée au moyen d'une unité d'étalonnage (4, 5, 6),
de préférence l'unité d'étalonnage (4, 5, 6) comprenant un corps d'étalonnage (4, 5) disposé de manière stationnaire dans le trajet de faisceau mesurable et/ou mesuré du tube à rayons X (10) et par rapport au tube à rayons X (10), au détecteur de rayons X (9) ou au tube à rayons X (10) et au détecteur de rayons X (9).

4. Système de capture d'image à rayons X selon la revendication précédente, **caractérisé en ce que** l'unité d'étalonnage (4, 5, 6) comprend un système de mesure de position (6), plus particulièrement une caméra de position et/ou une caméra de système de navigation, avec lequel la position et/ou l'orientation dans l'espace du corps d'étalonnage (4, 5) pour la détermination de données de position, qui sont nécessaires pour le calcul des images superposées d'une reconstitution d'images superposées, peut être mesurée et avec lequel ces données de position obtenues à partir d'une mesure du corps d'étalonnage (4, 5) dans une position et/ou une orientation définie dans l'espace peuvent être enregistrées, et/ou peuvent être transmises pour l'enregistrement, de préférence pour l'enregistrement dans l'unité de mémoire (1a), en tant que données d'étalonnage en même temps que l'orientation du tube à rayons X (10) et du détecteur de rayons x (9) par rapport à la direction de référence (R) prédéfinie, déterminée au moment de cette mesure au moyen du capteur de position (2).

5. Système de capture d'image à rayons X selon la revendication précédente, **caractérisé en ce que** les données de position nécessaires pour le calcul des images superposées de la reconstitution peuvent être extraites avec l'unité de calcul (1b) à l'aide des orientations momentanées enregistrées correspondantes des images de projection à rayons X à utiliser pour la reconstitution à partir des données d'étalonnage à l'aide d'un procédé d'interpolation, plus particulièrement au moyen d'une interpolation basée sur une spline.

6. Système de capture d'image à rayons X selon l'une des revendications précédentes, **caractérisé par** exactement une direction de référence (R) prédéfinie.

7. Système de capture d'image à rayons X selon l'une des revendications précédentes, **caractérisé par** une unité d'indication (12) avec laquelle, sur la base des orientations momentanées correspondantes des images de projection à rayons X déjà capturées, d'autres orientations du tube à rayons X (10) et du détecteur de rayons x (9) peuvent être calculées et indiquées, dans lesquelles des images de projection à rayons X doivent être capturées pour une utilisation pour une reconstitution suivante d'images superposées.

8. Système de capture d'image à rayons X selon la revendication précédente, **caractérisé en ce que** les autres orientations peuvent être calculées à partir de données de position d'images de projection à rayons X déjà capturées.

9. Système de capture d'image à rayons X selon l'une des revendications précédentes, **caractérisé par** une unité d'affichage (3) pour l'affichage d'images de projection à rayons X et/ou d'images superposées d'une reconstitution d'images superposées effectuée sur la base d'images de projection à rayons X capturées et/ou par une unité en forme de C, dont l'arc en forme de C (8) peut tourner autour de deux axes (axe C et axe P) orientés de préférence perpendiculairement, à une extrémité en forme de C de laquelle le tube à rayons X (10) est fixé et à l'autre extrémité en forme de C de laquelle le détecteur de rayons X (9) est fixé.

10. Système de capture d'image à rayons X selon la revendication précédente, **caractérisé en ce que** le capteur de position (2) est fixé à l'arc en forme de C (8) de façon à ce qu'une liaison est établie entre le capteur de position (2), le tube à rayons X (10) et le détecteur de rayons X (9).

11. Procédé de capture d'image à rayons X pour la capture d'images de projection à rayons X et d'informations d'orientation pour des images de projection de rayons X,
un tube à rayons X (10) et un détecteur d'image à rayons X (9) disposé dans le trajet de faisceau des tubes à rayons X (10) étant déplacés, pour la capture d'images de projection à rayons X d'un objet (O) à représenter, qui a été disposé de manière stationnaire entre le tube à rayons X (10) et le détecteur de rayons X (9) dans la zone de représentation (B), qui sont disposés de manière stationnaire l'un par rapport à l'autre, au moins partiellement autour de la zone de représentation (B),
un capteur de position (2) étant disposé de manière stationnaire par rapport au tube de rayons X (10) et au détecteur de rayons X (9),
l'orientation momentanée du tube à rayons X (10) et du détecteur de rayons X (9) par rapport à une direction de référence (R) prédéfinie étant déterminée avec le capteur de position (2) au moment de la capture d'une image de projection à rayons X,
les images de projection à rayons X capturées étant enregistrées, en même temps que l'orientation momentanée correspondante du tube à rayons X (10) et du détecteur de rayons X (9) et
pour une reconstitution d'images superposées, basée sur plusieurs images de projection à rayons X capturées, pour les images de projection à rayons X capturées utilisées pour cette reconstitution, les données de position de ces images de projection à rayons X, qui sont nécessaires pour le calcul des images superposées de la reconstitution, étant calculées à partir des orientations momentanées enregistrées correspondantes, une conversion d'une orientation par rapport à la direction de référence (R) en données de position correspondantes nécessaires ayant lieu pour le calcul des images superposées de la reconstitution.

12. Procédé de capture d'images à rayons X selon la revendication précédente, **caractérisé en ce que**, après le montage du capteur de position (2) selon la revendication 11, un système de capture d'images à rayons X selon l'une des revendications 2 à 10 est utilisé pour la capture.
